(19) Europäisches Patentamt
European Patent Office
Office européen des brevets

(11) Veröffentlichungsnummer: **0 158 596**
**A2**

(12) # EUROPÄISCHE PATENTANMELDUNG

(21) Anmeldenummer: 85810142.1

(22) Anmeldetag: 29.03.85

(51) Int. Cl.⁴: C 07 D 317/46
A 61 K 31/335

(30) Priorität: 06.04.84 CH 1752/84

(43) Veröffentlichungstag der Anmeldung: 16.10.85 Patentblatt 85/42

(84) Benannte Vertragsstaaten: AT BE CH DE FR GB IT LI LU NL SE

(71) Anmelder: CIBA-GEIGY AG
Postfach
CH-4002 Basel(CH)

(72) Erfinder: Ballenegger, Marc Ernest, Dr.

CH-1188 Gimel(CH)

(72) Erfinder: Zbinden, Paul
Im Rainacker 6
CH-4108 Witterswil(CH)

(54) **Neue substituierte Benzodioxolderivate, Verfahren zu deren Herstellung und entsprechende pharmazeutische Zusammensetzungen.**

(57) Die Erfindung betrifft den linksdrehenden und rechtsdrehenden Antipoden des Racemates der 5-Chlor-6-(3-chlorbenzolsulfonyl)-1,3-benzodioxol-2-carbonsäure der Formel I

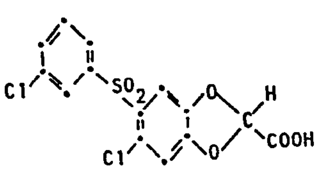

und deren Salze mit Basen und Niederalkylester des Racemates, sowie Verfahren zur Herstellung der obigen Verbindungen und ihre Salze und diese enthaltende pharmazeutischen Zusammensetzungen. Die neuen Stoffe besitzen diuretische und zusätzliche urikosurische und zum Teil auch hypolipidämische Wirksamkeiten und können, vorzugsweise in Form entsprechender pharmazeutischer Zusammensetzungen, zur Behandlung von Oedemen, der Hypertension und zum Teil auch als Lipidsenker verwendet werden.

EP 0 158 596 A2

0158596

CIBA-GEIGY AG
Basel (Schweiz)

4-14829/-

# Neue substituierte Benzodioxolderivate, Verfahren zu deren Herstellung und entsprechende pharmazeutische Zusammensetzungen

Die Erfindung betrifft neue Benzodioxolderivate, Verfahren zu deren Herstellung und entsprechende pharmazeutische Zusammensetzungen, sowie die Verwendung dieser neuen Stoffe und Zusammensetzungen.

Die Erfindung betrifft den linksdrehenden und rechtsdrehenden Antipoden des Racemates der 5-Chlor-6-(3-chlorbenzolsulfonyl)-1,3-benzodioxol-2-carbonsäure der Formel I

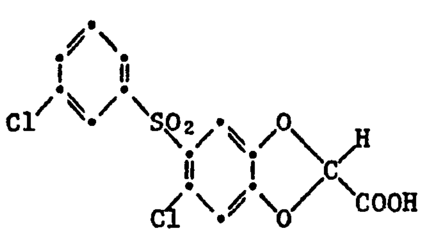

(I)

und deren Salze mit Basen. Die Erfindung betrifft auch die Niederalkylester des Racemates der 5-Chlor-6-(3-chlorbenzolsulfonyl)-1,3-benzodioxol-2-carbonsäure der Formel I.

Ein Niederalkylester enthält vorzugsweise bis zu 4 Kohlenstoffatome im Niederalkylrest und steht z.B. für einen Methyl-, gerade oder verzweigten Propyl- oder Butylester, insbesondere jedoch für einen Aethylester. Salze der neuen Verbindungen mit Basen, sind vor allem pharmazeutisch verwendbare Salze. Als solche Salze mit Basen kommen beispielsweise Alkalimetall- oder Erdalkalimetallsalze, wie Natrium-, Kalium-, Calcium,- oder Magnesiumsalze, ferner Ammoniumsalze mit Ammoniak oder organischen Aminen, wie Mono- oder Diniederalkylaminen, z.B. Methylamin, Aethylamin, Dimethylamin oder Diäthylamin, oder mit gegebenenfalls niederalkylierten Mono- oder Di(hydroxylalkyl)-aminen, oder mit Tri(hydroxyalkyl)aminen, z.B. 2-Aminoäthanol, 2-(Diäthylamino)-äthanol, 2,2'-Iminodiäthanol, N-Methyl-2,2'-iminodiäthanol oder 2,2',2"-Nitrilotri-äthanol, in Betracht.

Die neuen Verbindungen der allgemeinen Formel I und ihre Salze besitzen wertvolle pharmakologische Eigenschaften. Sie wirken insbesondere diuretisch und natriuretisch an der Ratte im Dosisbereich von 1 bis 300 mg/kg per os und am Hund in Dosen ab 1 bis 20 mg/kg per os, wie durch Sammeln des Harns während 3 Stunden nach Verabreichung (Ratte) bzw. stündlich während 5 Stunden nach Verabreichung (Hund) und Bestimmung des Harnvolumens und der Natrium-, Kalium- und Chlorionen festgestellt werden kann. Hierbei wird die Kalium-Ausscheidung weniger stark gesteigert als die Natrium Ausscheidung; hervorzuheben ist auch die gute Verträglichkeit.

Beispielsweise wird durch die Verabreichung von 10 mg/kg per os (-)-Chlor-6-(3-chlorbenzolsulfonyl)-1,3-benzodioxol-2-carbonssäure an Ratten (6 Tiere pro Dosis) im Vergleich zu unbehandelten Kontrolltieren die Ausscheidung von Natriumionen auf das 5,7-fache, von Kaliumionen auf das 2,7-fache und von Chlorionen auf das 10,1-fache gesteigert.

Die Steigerung der Kaliumionenausscheidung durch die Verbindungen der Formel I beim Hund fällt in analoger Weise wie bei der Ratte im Vergleich zur Steigerung der Natriumionen- und Chlorionenausscheidung auch nur sehr gering aus.

Weiter besitzen die Verbindungen der allgemeinen Formel I uricosurische Wirksamkeit, wie sich z.B. anhand von Versuchen am Cebus-Affen (Cebus apella) zeigen lässt. Bei diesen Versuchen wird den Versuchstieren in Pentobarbital-Narkose Polyfructosan in Ringer-Lösung intravenös infundiert und die Prüfsubstanz als wässrige Lösung in ansteigenden Dosen intravenös injiziert. Vor der ersten Prüfsubstanz-Verabreichung und nach jeder Prüfsubstanzdosis wird während je drei 10-minütigen Perioden der Harn gesammelt und vor der ersten und nach der letzten Sammelperiode jeweils arteriell Blut entnommen. Die Harnsäure- und Polyfructosan-clearance wird aus deren Plasma- und Urin-Konzentration berechnet und schliesslich die fraktionelle Harnsäureausscheidung (Fractional excretion of uric acid, $FE_{UR}$) als Quotient von Harnsäureclearance und glomerulärer Filtrationsrate bestimmt. Verbindungen der allgemeinen Formel I, zeigen in diesem Test Wirkungen im Dosisbereich von 1 bis 10 mg/kg i.v.. Entsprechend können die Verbindungen der allgemeinen Formel I und ihre pharmazeutisch annehmbaren Salze als kaliumschonende Diuretika mit urikosurischer Zusatzwirkung, z.B. zur Behandlung von Oedemen und der Hypertension, verwendet werden.

Der rechtsdrehende Antipode des Racemates, die (+)5-Chlor-6-(3-chlorbenzolsulfonyl)-1,3-benzodioxol-2-carbonsäure besitzt insbesondere noch zusätzlich hypolipidämische Wirkungen, wobei die Senkung von "low-density" Lipoprotein (LDL) und die Senkung von Cholesterin und Triglyceriden im Serum bei peroraler Verabreichung an der Ratte nachgewiesen werden kann. Die obengenannte Verbindung kann daher als Lipidsenker verwendet werden.

Die Erfindung betrifft in erster Linie die (-)-5-Chlor-6-(3-chlorbenzolsulfonyl)-1,3-benzodioxol-2-carbonsäure und ihre Salze mit Basen.

Die neuen Verbindungen, Antipoden des Razemates und ihre Salze der Verbindungen der allgemeinen Formel I, und die Niederalkylester des Razemates solcher Verbindungen werden in an sich bekannter Weise hergestellt, indem man

a) eine Verbindung der Formel II

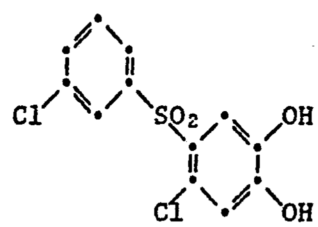

(II)

oder einem Salz davon, mit einer Verbindung der allgemeinen Formel III,

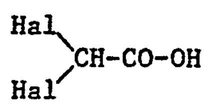

(III)

oder einem Niederalkylester oder einem Salz davon, in welcher Hal Halogen bedeutet, umsetzt, oder

b) in einer Verbindung der Formel IV

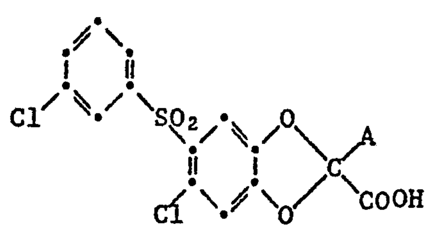

(IV)

einem Niederalkylester oder Salz davon, in welcher A Carboxy, Niederalkoxycarbonyl oder Acetyl bedeutet, den Rest A durch Wasserstoff ersetzt, oder

c) zur Herstellung eines Niederalkylesters einer Verbindung der Formel I eine Verbindung der allgemeinen Formel V

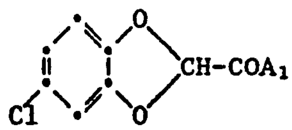

(V)

in welcher $A_1$ Niederalkoxy bedeutet, mit einer Verbindung der Formel VI

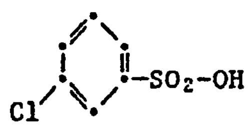

(VI)

oder einem Salz oder Anhydrid desselben umsetzt, oder

d) eine gegebenenfalls <u>in situ</u> gebildete Metallverbindung der allgemeinen Formel VII oder VIII

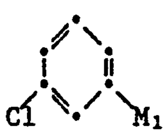

(VII) oder

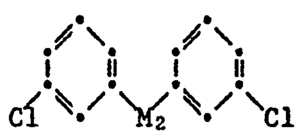

(VIII)

in welcher $M_1$ ein einwertiges bzw. $M_2$ ein zweiwertiges Metallion bedeutet, mit einer Verbindung der Formel IX

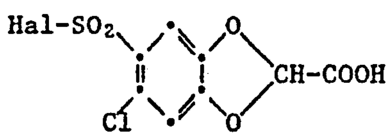

(IX)

oder einem Niederalkylester oder Salz davon, umsetzt, oder

e) zur Herstellung einer Verbindung der Formel I oder eines Salzes dieser Verbindung in einer Verbindung der Formel X,

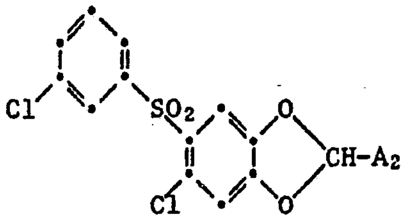

(X)

in welcher $A_2$ eine in die Carboxygruppe überführbare Gruppe bedeutet, die Gruppe $A_2$ in die Carboxygruppe in freier oder Salzform überführt, oder

f) eine Verbindung der Formel XI

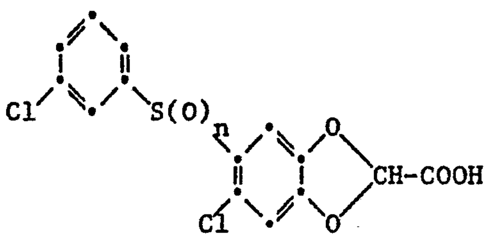

(XI)

in welcher n die Bedeutung von 0 oder 1 hat, zu einer Verbindung der Formel I, in der n die Zahl 2 bedeutet, oxydiert und gegebenenfalls eine erhaltene Verbindung der Formel I in einen Niederalkylester überführt und/oder eine erhaltene Verbindung der Formel I in die optischen Antipoden zerlegt, und/oder eine erhaltene freie Verbindung in ein Salz mit einer Base überführt oder eine solche Verbindung aus einem erhaltenen Salz freisetzt.

In den Ausgangsstoffen der allgemeinen Formel III ist Hal vorzugsweise Chlor oder Brom, kann aber auch Fluor oder Jod sein, wobei auch zwei unter sich verschiedene Halogenatome vorliegen können. Die Umsetzungen gemäss Verfahren a) werden vorzugsweise in unter den Reaktionsbedingungen inerten organischen Lösungsmitteln, beispielsweise in ätherartigen, wie z.B. Dibutyläther, 1,2-Dimethoxyäthan, Diäthylenglykoldimethyläther, Tetrahydrofuran oder Dioxan; alkoholartigen, wie z.B. Methanol, Aethanol, Isopropanol, Butanol, 2-Methoxyäthanol oder 2-Aethoxyäthanol; oder amidartigen, wie z.B. Dimethylformamid oder N,N,N',N',N",N"-Hexamethylphosphorsäuretriamid; oder in Kohlenwasserstoffen, wie z.B. Petroläther, Cyclohexan, Benzol oder Toluol durchgeführt. Umsetzungen mit freien Verbindungen der allgemeinen Formel II wie auch mit freien Halogenessigsäuren der allgemeinen Formel III werden vorzugsweise in Gegenwart von basischen Stoffen durchgeführt. Als solche können beispielsweise organische oder anorganische Derivate von Alkali- oder Erdalkalimetallen, als organische Derivate z.B. Alkalimetall- oder Erdalkalimetallalkoxide, wie Natrium- oder Lithiummethoxid, -äthoxid, -n-butoxid oder -tert.-butoxid, bzw. Barium-methoxid, oder als anorganische Derivate z.B. entsprechende Hydroxide, wie Natrium-, Kalium- oder Calciumhydroxid, oder Carbonate, wie z.B. Natrium- oder Kaliumcarbonat, verwendet werden. Insbesondere Carbonate können in grösserem, z.B. bis fünffachem Ueberschuss eingesetzt werden. Bei Verwendung von Carbonaten können auch noch weitere organische Lösungsmittel, wie Niederalkanone, z.B. Aceton oder 2-Butanon, als genügend inert in Betracht kommen.

Als Salze von Verbindungen der allgemeinen Formel II und von gegebenenfalls verwendeten, unter die allgemeine Formel III fallenden Dihalogenessigsäuren eignen sich beispielsweise entsprechende Alkalimetallsalze oder Erdalkalimetallsalze. Die Reaktionstemperaturen liegen beispielsweise zwischen Raumtemperatur und etwa 150°C und vorzugsweise zwischen etwa 70 und 120°C.

Eine Anzahl Ausgangsstoffe der allgemeinen Formeln II und III sind bekannt und weitere analog zu den bekannten Verbindungen herstellbar. So kann man beispielsweise Ausgangsstoffe der allgemeinen Formel II herstellen, indem man zunächst 1,2-Dimethoxybenzol, das entsprechend der Definition durch Chlor substituiert ist, mit einer Sulfonsäure in Polyphosphorsäure in der Wärme, z.B. bei ca. 100-110°C zum entsprechenden Sulfon kondensiert oder mit einem von

einer solchen Sulfonsäure oder auch einer entsprechenden Sulfonsäure abgeleiteten Acylhalogenid nach Friedel-Crafts, z.B. mittels Aluminiumchlorid in 1,2-Dichloräthan bei Raumtemperatur, ebenfalls zum entsprechenden Sulfon kondensiert und in diesem die beiden Methoxygruppen in an sich bekannter Weise, z.B. durch Erhitzen mit Pyridin-hydrochlorid oder mit 48%iger Bromwasserstoffsäure in Essigsäure, spaltet.

Zur Durchführung des Verfahrens b) erhitzt man beispielsweise einen Ausgangsstoff der allgemeinen Formel IV, in welchem A Carboxy bedeutet in An- oder Abwesenheit eines Katalysators, z.B. Kupferpulver, und/oder eines Lösungs- oder Verdünnungsmittels, wie z.B. o-Dichlorbenzol oder 1,2,3,4-Tetrahydronaphthalin, bis die mindestens annährend äquimolare Menge Kohlendioxid freigesetzt ist. Ausgangsstoffe der allgemeinen Formel IV, in denen A Carboxy bedeutet, werden beispielsweise durch Hydrolyse von entsprechenden Verbindungen , in denen die Carboxygruppe durch Niederalkyl verestert ist und als bzw. anstelle von A Niederalkoxycarbonyl oder Cyano steht, in saurem oder alkalischem Medium, beispielsweise durch Erwärmen mit einer starken Mineralsäure in wässrigem oder wässrigorganischen, z.B. wässrig-niederalkanolischem Medium, bzw. mit der mindestens doppelmolaren Menge eines Alkalimetallhydroxids, insbesondere Natrium- oder Kaliumhydroxid, z.B. in einem Niederalkanol, wie Methanol, Aethanol, Isopropanol oder n-Butanol, oder in einem Niederalkandiol oder Monoalkyläther desselben, z.B. Aethylenglykol, 2-Methoxyäthanol oder 2-Aethoxyäthanol, hergestellt, wobei den genannten Lösungsmitteln gegebenenfalls Wasser im Volumenverhältnis von Lösungsmittel zu Wasser von ca. 10:1 bis 1:2 zugefügt wird. Ferner kann als Reaktionsmedium auch Wasser z.B. ein Gemisch von Wasser mit wasserlöslichen, ätherartigen Lösungsmitteln, wie Dioxan oder Tetrahydrofuran, verwendet werden.

Erfolgt die Hydrolyse in einer wasserhaltigen Mineralsäure, so kann die verfahrensgemässe Decarboxylierung daran anschliessend, d.h. im gleichen Medium und Arbeitsgang durchgeführt werden.

Ausgangsstoffe der allgemeinen Formel IV in denen A Carboxy bedeutet und die andere Carboxygruppe durch einen Niederalkanol verestert ist, kann man z.B. durch partielle Hydrolyse in alkalischem Medium aus entsprechenden Verbindungen mit Niederalkoxycarbonyl als Rest A unter Verwendung der etwa äquimolaren Menge eines Alkalimetallhydroxids anstelle der mindestens doppeltmolaren Menge herstellen. Eine andere Möglichkeit zur Herstellung solcher Ausgangsstoffe der allgemeinen Formel IV besteht in der Hydrogenolyse von entsprechenden Verbindungen, in denen sich anstelle von A Benzyloxycarbonyl befindet.

Die Desalkoxycarbonylierung oder Desacetylierung von entsprechenden Niederalkylestern als Ausgangsstoffen der allgemeinen Formel IV, d.h. solche, in denen A Niederalkoxycarbonyl oder Acetyl bedeutet, erfolgt beispielsweise durch Umsetzung mit der etwa äquimolaren Menge eines Alkalimetallniederalkoxides in einem wasserfreien Niederalkanol, wobei, vorzugsweise dasselbe niedere Alkanol, z.B. Methanol, Aethanol, n-Butanol, als Komponente des Ausgangsesters und des Niederalkoxids wie auch als Reaktionsmedium gewählt wird. Man kann aber auch durch Verwendung eines relativ höhersiedenden, mit dem als Esterkomponente vorliegenden Niederalkanol nicht identischen Alkanols als Reaktionsmedium und Abdestillieren eines Teiles davon gleichzeitig mit der definitionsgemässen Umsetzung eine Umesterung durchführen oder aber eine partielle Umesterung in Kauf nehmen, falls der als Reaktionprodukt anfallende Ester der allgemeinen Formel I nicht direkt als Wirkstoff verwendet, sondern zur entsprechenden Säure hydrolysiert werden soll. Ferner kann als Reaktionsmedium anstelle eines niederen Alkanols z.B. auch ein inertes organisches Lösungsmittel, wie z.B. Benzol oder Toluol, verwendet werden. Die definitionsgemässe Umsetzung wird bei Raumtemperatur oder erhöhter Temperatur, z.B. bei Siedetemperatur des verwendeten Reaktionsmediums, durchgeführt. Gegebenenfalls kann der entstandene Ester der allgemeinen Formel I, wie bereits im Zusammenhang mit der Umesterung erwähnt, im gleichen Arbeitsgang zur entsprechenden Säure hydrolysiert werden, wenn man dem Reaktionsmedium Wasser zufügt.

Die Ausgangsstoffe der allgemeinen Formel IV, in denen A Niederalkoxycarbonyl oder Acetyl ist, sowie die weiter oben genannten Vorprodukte zu Verbindungen der allgemeinen Formel IV mit Carboxy als Rest A, die als bzw. anstelle von A Niederalkoxycarbonyl bzw. Cyano enthalten, können analog Verfahren a) durch Umsetzung von Verbindungen der allgemeinen Formel II mit geminalen Dihalogenverbindungen, die sich von solchen der allgemeinen Formel III durch das Vorliegen von Niederalkoxycarbonyl, Acetyl oder Cyano anstelle des neben beiden Halogenatomen befindlichen Wasserstoffatoms unterscheiden, in Gegenwart einer Base hergestellt werden.

Gemäss Verfahren c) kann man beispielsweise freie Sulfonsäuren der allgemeinen Formel VI in Polyphosphorsäure oder Pyrophosphorsäure unter Erwärmen, z.B. bei etwa 80 bis etwa 120°C, insbesondere bei etwa 100 bis 110°C mit Verbindungen der allgemeinen Formel V kondensieren. Weiter kann man Salze der allgemeinen Formel VI, beispielsweise deren Halogenide, wie Chloride oder Bromide, ferner z.B. deren symmetrische Anhydride, in Gegenwart üblicher Friedel-Crafts-Kondensationsmittel, wie Aluminiumchlorid oder Zinn(IV)-chlorid, ferner z.B. Zinkchlorid, weiter z.B. in konz. Schwefelsäure, Phosphorsäure, Polyphosphorsäure oder Pyrophosphorsäure mit Verbindungen der allgemeinen Formel V kondensieren. Die vorgenannten Säuren werden bevorzugt verwendet, wenn man als Ausgangsstoff ein symmetrisches Anhydrid einer Sulfonsäure der allgemeinen Formel VI einsetzt. Die Umsetzungen werden bei Verwendung von Kondensationsmitteln vorzugsweise in einem Lösungsmittel vorgenommen. Als solche kann man beispielsweise Halogenkohlenwasserstoffe, wie 1,2-Dichloräthan, Tetrachlorkohlenstoff, Methylenchlorid, o-Dichlorbenzol, weiter z.B. aliphatische oder cycloaliphatische Kohlenwasserstoffe, wie Heptan oder Cyclohexan, Nitrokohlenwasserstoffe, wie Nitromethan, Nitrocyclohexan oder Nitrobenzol und ferner unter milden Bedingungen auch Schwefelkohlenstoff verwenden. Die Reaktionstemperatur liegt zwischen etwa -20° und +80°C, vorzugsweise zwischen etwa 0° und Raumtemperatur.

Die Ausgangsstoffe der allgemeinen Formel V können ihrerseits analog Verfahren a) aus einem Chlor-substituierten 1,2-Benzendiol, wie z.B. 4-Chlor-1,2-benzendiol mit Dihalogenessigsäuren oder deren funktionellen Derivaten entsprechend der allgemeinen Formel III hergestellt werden. Die als zweite Reaktionskomponente benötigte Sulfonsäure der allgemeinen Formel VI und ihre funktionellen Derivate sind bekannt.

Im Verfahren gemäss d) verwendet man als Ausgangsstoffe der allgemeinen Formel VII z.B. durch Chlor substituiertes Phenyllithium, vgl. z.B. A. Schönberg et al. Chem. Ber. 66 237-244 (1933), oder auch entsprechende Grignardverbindungen, bzw. als Ausgangsstoff der allgemeinen Formel VIII durch Chlor substituiertes Diphenylcadmium, vgl. z.B. H.R. Henze et al., J. Chem. Soc. 1957, 1410-1413 oder durch Chlor substituiertes Diphenylquecksilber, und arbeitet in beiden Fällen z.B. in abs. Diäthyläther oder einem anderen ätherartigen Lösungsmittel. Zur Ausgangsverbindung der Formel IX gelangt man z.B. durch Umsetzung der entsprechenden Verbindung der Formel V mit Chlorsulfonsäure zur entsprechenden Chlorsulfonylverbindung.

Bei Verbindungen der Formel X gemäss Verfahren c) kann die Umwandlung einer Gruppe $A_2$ in die Carboxygruppe in an sich bekannter Weise, insbesondere durch Hydrolyse in alklischem oder saurem Medium erfolgen, wobei man im ersteren Fall auch direkt ein Salz erhalten kann. Ausgangsstoffe für die Hydrolyse sind zunächst solche Verbindungen der Formel X, in denen $A_2$, insbesondere leicht hydrolysierbare Reste, wie z.B. die Niederalkylester, im weiteren aber auch andere funktionelle Derivate der als Endstoffe gewünschten Carbonsäuren, wie z.B. Nitrile und Imidoester, insbesondere Imidoniederalkylester, von unter die allgemeine Formel I fallenden Carbonsäuren. Die Hydrolyse erfolgt beispielsweise in niederalkanolischen oder wässrig-niederalkanolischen Alkalihydroxidlösungen bei Raumtemperatur bis etwa 100°C bzw. Siedetemperatur des Reaktionsmediums. Niederalkylester, wie Methyl- oder Aethylester und andere leicht spaltbare Ester von unter die allgemeine Formel I fallenden Carbonsäuren können unter noch milderen Bedingungen, z.B. in

Gegenwart von Kalium- oder Natriumcarbonat bei Raumtemperatur oder nötigenfalls schwach erhöhten Temperaturen von z.B. 40°C, in wässerig-organischem Medium, z.B. in dem beim Versetzen des bei der Umsetzung gemäss a) erhaltenen Reaktionsgemisches in einem mit Wasser mischbaren Lösungsmittel, wie z.B. 1,2-Dimethoxyäthan, mit Wasser hydrolysiert werden. Aus den dabei zunächst erhaltenen Alkalimetallsalzlösungen der unter die allgemeine Formel I fallenden Carbonsäuren kann man entweder durch Einengen und Abfiltrieren bzw. Eindampfen und Umkristallisieren direkt das entsprechende reine Alkalisalz gewinnen oder zunächst die Carbonsäure freisetzen, anschliessend z.B. durch Umkristallisation reinigen und gewünschtenfalls wiederum in ein Salz mit einer geeigneten anorganischen oder organischen Base überführen. Funktionelle Derivate der unter die Formel I fallenden Carbonsäuren lassen sich ferner auch in saurem Medium, z.B. durch Erwärmen in einer mit Wasser verdünnten, z.B. 60-70%igen Schwefelsäure oder in niederalkanolisch-wässeriger Salzsäure, in die freie Carbonsäure der Formel I überführen.

Die benötigten funktionellen Derivate von Carbonsäuren, welche unter die Formel I fallen, werden z.B. gemäss einem der vorgenannten Verfahren, und andere funktionelle Derivate, wie z.B. Nitrile, analog zu diesen Verfahren hergestellt.

Die Umsetzung der freien Carbonsäuren der Formel I mit gegebenenfalls reaktionsfähigen Estern und Niederalkanolen, wie z.B. Halogeniden oder organischen Sulfonsäureestern, wie z.B. Niederalkansulfonsäure- oder Arensulfonsäureestern, wie Methansulfonsäure- bzw. p-Toluolsulfonsäureestern wird vorteilhaft in Gegenwart eines sauren wasserabspaltenden Katalysators, wie einer Protonensäure, z.B. von Chlor- oder Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Niederalkanols und/oder in einem inerten Lösungsmittel, z.B. in einem Kohlenwasserstoff der Benzolreihe, wie Benzol oder Toluol, einem halogenierten Kohlenwasserstoff wie Chloroform, Methylenchlorid oder Chlorbenzol, oder in einem ätherartigen

Lösungsmittel, wie Tetrahydrofuran, erforderlichenfalls unter destillativer, z.B. azeotroper, Entfernung des bei der Reaktion freigesetzten Wassers durchgeführt. Weiter kann man die Umsetzungen auch in Gegenwart anderer wasserbindender Kondensationsmittel, z.B. von durch Kohlenwasserstoffreste substituierten Carbodiimiden, wie N,N'-Diäthyl-, N,N'Dicyclohexyl- oder N-Aethyl-N'-(3-dimethylaminopropyl)-carbodiimid, in inerten organischen Lösungsmitteln, z.B. den vorgenannten durchführen.

Zu Umesterungen von Niederalkylestern von Carbonsäuren der allgemeinen Formel I verwendet man vorzugsweise Niederalkanole mit deutlich über demjenigen des veresterten Niederalkanols liegenden Siedepunkt und führt die Reaktion z.B. in einem Ueberschuss des Niederalkanols und/oder einem inerten organischen, vorzugsweise ebenfalls deutlich über dem Niederalkanol siedenden Lösungsmittel, vorzugsweise in Gegenwart eines Katalysators, z.B. eines Alkalimetall-niederalkoxids, wie Natrium- oder Kalium-methoxid oder -äthoxid, in der Wärme und vorzugsweise unter Abdestillieren des freigesetzten Niederalkanols durch.

Die Oxidation gemäss Verfahren b) erfolgt beispielsweise mittels Wasserstoffperoxid in einem gegenüber diesem inerten organischen oder organisch-wässrigen Lösungsmittel, wie gegebenenfalls Wasser enthaltender Essigsäure, z.B. in dem aus Eisessig und wässriger Wasserstoffperoxidlösung entstehenden Gemisch, bei mässig erhöhten Temperaturen zwischen etwa 60 und 100°C, insbesondere bei etwa 80-90°C, und mit der mehr als doppeltmolaren Menge Wasserstoffperoxid, wenn als Oxidationsprodukt eine Sulfonylverbindung erhalten werden soll.

Eine erhaltene salzbildende Verbindung der Formel I kann in an sich bekannter Weise in Salz übergeführt werden, z.B. mit entsprechenden Basen, wie z.B. Alkalimetallhydroxiden, in Salze mit Basen. Vorzugsweise werden pharmazeutisch annehmbare Salze hergestellt.

Erhaltene Salze können in an sich bekannter Weise in die freien Verbindungen umgewandelt werden, z.B. durch Behandeln mit einem sauren Reagens, wie einer Mineralsäure.

Die Verbindungen einschliesslich ihrer Salze können auch in Form ihrer Hydrate erhalten werden, oder ihre Kristalle können das zur Kristallisation verwendete Lösungsmittel einschliessen.

Infolge der engen Beziehung zwischen den neuen Verbindungen der allgemeinen Formel I, in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die neuen Verbindungen können, je nach der Anzahl der Asymmetriezentren sowie auch nach der Wahl der Ausgangsstoffe und Arbeitsweisen, in Form von Racematen oder Racematgemischen (Diastereomerengemischen) oder gegebenenfalls auch als reine Antipoden erhalten werden.

Erhaltene Racematgemische können auf Grund der physikalischchemischen Unterschiede der Bestandteile in bekannter Weise in die reinen Racemate bzw. Diastereomeren aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes der allgemeinen Formel I mit einer mit der racemischen Säure Salze bildenden optischaktiven Base, und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

Die Erfindung betrifft im weiteren pharmazeutische Präparate, die Verbindungen der allgemeinen Formel I als Wirkstoffe enthalten, sowie Verfahren zu ihrer Herstellung.

Bei den erfindungsgemässen pharmazeutischen Präparaten handelt es sich um solche zur enteralen, wie oralen oder rektalen, sowie zur parenteralen Verabreichung an Warmblüter. Die Dosierung des Wirkstoffs, der alleine oder zusammen mit dem üblichen Träger- und Hilfsmaterial appliziert wird, hängt von der Warmblüterspezies, deren Alter und dem individuellen Zustand sowie der Applikationsweise ab. Die täglichen Dosen bewegen sich zwischen 0,15 und 20 mg/kg für Mammalien und liegen für solche von etwa 70 kg Gewicht je nach individuellem Zustand und Alter vorzugsweise zwischen 10 und 600 mg, insbesondere zwischen 25 und 300 mg. Entsprechende orale Doseneinheitsformen, z.B. Dragées oder Tabletten oder Kapseln, enthalten vorzugsweise 5 bis 150 mg, insbesondere 10 bis 100 mg eines erfindungsgemässen Wirkstoffes, d.h. einer Verbindung der allgemeinen Formel I oder eines pharmazeutisch annehmbaren Salzes einer zur Salzbildung befähigten Verbindung der allgemeinen Formel I, zusammen mit pharmazeutischen Trägerstoffen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelaltine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster LInie Fliessregulier- und Schmiermittel, z.B. Kieselsäure, Talk, Stearinsäure oder Salze davon, wie Magnesium- oder Calciumstearat, und/oder Polyäthylenglykol. Drageée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten. Lacklösungen in geeigneten organische Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magensiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als reaktal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eigenen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eigenen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die Erfindung betrifft ebenfalls die Verwendung der neuen Verbindungen der Formel I und der pharmazeutisch annehmbaren Salze von solchen als pharmakologisch aktive Verbindungen, insbesondere als Diuretika mit urikosurischer Zusatzwirkung, bzw. als Lipidsenker im einzelnen spezifischen Falle, vorzugsweise in Form von pharmazeutischen Präparaten in einem Verfahren zur prophylaktischen und/oder therapeutischen Behandlung des tierischen oder menschlichen Körpers, insbesondere zur Behandlung von Oedemen und/oder der Hypertenison oder auch zur Verwendung als Hypolipidämika.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Beispiel 1: 15,9 g (0,05 Mol) 4-Chlor-5-(3-chlorbenzolsulfonyl)-1,2-benzendiol, 27,5 g (0,2 Mol) Kaliumkarbonat und 9,67 g (0,075 Mol) Dichloressigsäure werden in 125 ml Dimethylformamid während 6 Stunden bei 100°C gerührt. Man dampft am Rotationsverdampfer unter Wasserstrahlvakuum das Dimethylformamid ab und nimmt den Rückstand in Wasser auf. Mit konzentrierter Salzsäure wird auf den pH-Wert von 1-2 gestellt und die Rohsäure mit Aethylacetat extrahiert. Die Extraktlösung wird eingedampft und der Rückstand in 150 ml abs. Aethanol gelöst. Nach Zusatz von 0,5 ml konzentrierter Salzsäure erhitzt man für 1 Stunde am Rückfluss. Man engt auf ca. 50 ml Volumen ein und lässt das Reaktionsgemisch abkühlen. Aus der braunen Lösung kristallisiert der 5-Chlor-6-(3-chlorbenzolsulfonyl)-1,3-benzodioxol-2-carbonsäureäthylester als farblose Kristalle vom Smp. 97-98°C aus.

Nach Freisetzung mit 2-n NaOH und Isolierung der 5-Chlor-6-(3-chlorbenzolsulfonyl)-1,3-benzodioxol-2-carbonsäure wird diese aus Toluol/Aethylacetat umkristallisiert; Smp. 182-184.

Beispiel 2: 35,5 g (0,1 Mol) 5-Chlor-6-(3-chlorbenzolsulfonyl)-1,3-benzodioxol-2-carbonsäure, erhalten aus dem Ester gemäss Beispiel 1 werden in 110 ml Acetonitril gelöst und man versetzt in der Wärme mit einer Lösung von 6,7 g (0,055 Mol) S(-)-1-Phenyläthylamin und 4,6 g (0,045 Mol) Triäthylamin in 30 ml Acetonitril. Die beim Erkalten der Lösung ausgeschiedenen Kristalle werden abfiltriert und 2 mal aus Methanol umkristallisiert. Man erhält das S(-)-1-Phenyläthylaminsalz der (-)-5-Chlor-6-(3-chlorbenzolsulfonyl)-1,3-benzdioxol-2-carbonsäure vom Smpl 209-210. Nach

Freisetzung der Säure und Umkristallisation aus Toluol erhält man die (-)-5-Chlor-6-(3-chlorbenzolsulfonyl-)-1,3-benzodioxol-2-carbonsäure vom Smp. 151-153°C, $[\alpha]_D^{20}$ -7° (c = 1, Aceton).

Beispiel 3: Beim analogen Vorgehen wie unter Beispiel 2 erhält man unter Verwendung von R(+)-1-Phenyläthylamin als optisch aktive Hilfsbase nach Salzbildung, Umkristallisation und Freisetzung der Säure die (+)-5-Chlor-6-(3-chlorbenzolsulfonyl)-1,3-benzodioxol-2-carbonsäure vom Smp. 151-153°C, $[\alpha]_D^{20}$ +7°C (c = 1, Aceton).

Beispiel 4: Tabletten enthaltend 50 mg Wirkstoff können beispielsweise in folgender Zusammensetzung hergestellt werden:

| Zusammensetzung | pro Tablette |
|---|---|
| Wirkstoff | 50 mg |
| Milchzucker | 50 mg |
| Weizenstärke | 73 mg |
| Kolloidale Kieselsäure | 13 mg |
| Talk | 12 mg |
| Magnesiemstearat | 2 mg |
| | 200 mg |

Herstellung

Der Wirksktoff wird mit Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Die Masse wird durch ein Sieb von ca. 3 mm Maschenweite getrieben, getrockent und das trockene Granulat nochmals durch ein Sieb getrieben. Darauf werden die restliche Weizenstärke, Talk und Magnesiumstearat zugemischt. Die erhaltene Mischung wird zu Tabletten von 200 mg mit Bruchkerbe(n) verpresst.

Beispiel 5: Um 1000 Kapseln mit je 50 mg Wirkstoffgehalt herzustellen, mischt man 50,0 g Wirkstoff mit 223,0 g Lactose, befeuchtet die Mischung gleichmässig mit einer wässrigen Lösung von 2,0 g Gelatine und granuliert sie durch ein geeignetes Sieb (z.B. Sieb III nach Ph. Helv. V.). Das Granulat mischt man mit 10,0 g getrockneter Maisstärke und 15,0 g Talk und füllt es gleichmässig in 1000 Hartgelatinekapseln der Grösse 1.

Patentansprüche für die Vertragsstaaten: DE, GB, FR, CH, LI, IT, NL, BE, SE, LU

1. Der linksdrehende und rechtsdrehende Antipode des Racemates der 5-Chlor-6-(3-chlorbenzolsulfonyl)-1,3-benzodioxol-2-carbonsäure der Formel I

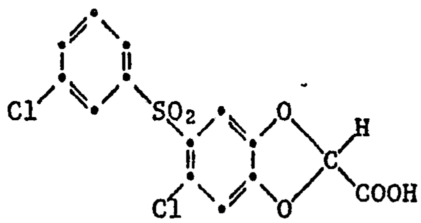

und deren Salze mit Basen und Niederalkylester des Racemates.

2. (-)-5-Chlor-6-(3-chlorbenzolsulfonyl)-1,3-benzodioxol-2-carbonsäure und ihre pharmazeutisch annehmbaren Salze mit Basen.

3. (+)-5-Chlor-6-(3-chlorbenzolsulfonyl)-1,3-benzodioxol-2-carbonsäure und ihre pharmazeutisch annehmbaren Salze mit Basen.

4. 5-Chlor-6-(3-chlorbenzolsulfonyl)-1,3-benzodioxol-2-carbonsäureäthylester.

5. Pharmazeutische Präparate, enthaltend eine Verbindung gemäss einem der Ansprüche 1-4 oder ein pharmazeutisch annehmbares Salz einer zur Salzbildung befähigten Verbindung gemäss einem dieser Ansprüche.

6. Verbindungen gemäss einem der Ansprüche 1-4 und pharmazeutisch annehmbare Salze von zur Salzbildung befähigten Verbindungen gemäss einem dieser Ansprüche zur Anwendung in einem Verfahren zur therapeutischen Behandlung des tierischen oder menschlichen Körpers.

7. Verbindungen gemäss einem der Ansprüche 1-4 und pharmazeutisch annehmbare Salze von zur Salzbildung befähigten Verbindungen gemäss einem dieser Ansprüche als Diuretika mit urikosurischer Zusatzwirkung.

8. Die (+)-5-Chlor-6-(3-chlorbenzolsulfonyl)-1,3-benzodioxol-2-carbonsäure und ihre pharmazeutisch annehmbaren Salze mit Basen als Hypolipidämika.

9. Verwendung von Verbindungen gemäss einem der Ansprüche 1-4 oder von pharmazeutisch annehmbaren Salzen von zur Salzbildung befähigten Verbindungen gemäss einem dieser Ansprüche zur Herstellung von Arzneimitteln auf nicht-chemischem Wege.

10. Verfahren zur Herstellung der im Anspruch 1 beanspruchten Verbindungen der Formel I, dadurch gekennzeichnet, dass man

a) eine Verbindung der Formel II

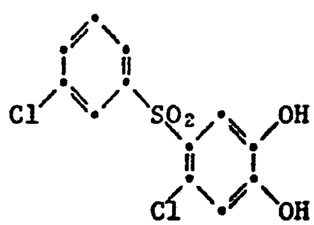

(II)

oder einem Salz davon, mit einer Verbindung der allgemeinen Formel III,

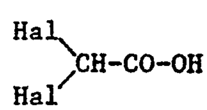

(III)

oder einem Niederalkylester oder einem Salz davon, in welcher Hal Halogen bedeutet, umsetzt, oder

b) in einer Verbindung der Formel IV

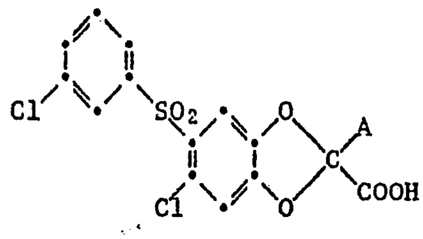

(IV)

einem Niederalkylester oder Salz davon, in welcher A Carboxy, Niederalkoxycarbonyl oder Acetyl bedeutet, den Rest A durch Wasserstoff ersetzt, oder

c) zur Herstellung eines Niederalkylesters einer Verbindung der Formel I eine Verbindung der allgemeinen Formel V

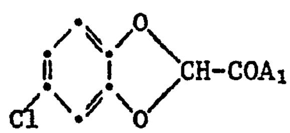

(V)

in welcher $A_1$ Niederalkoxy bedeutet, mit einer Verbindung der Formel VI

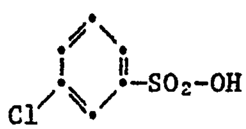

(VI)

oder einem Salz oder Anhydrid desselben umsetzt, oder

d) eine gegebenenfalls <u>in situ</u> gebildete Metallverbindung der allgemeinen Formel VII oder VIII

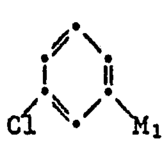

(VII) oder

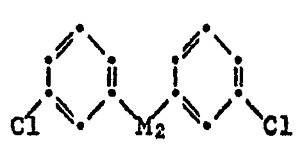

(VIII)

in welcher $M_1$ ein einwertiges bzw. $M_2$ ein zweiwertiges Metallion bedeutet, mit einer Verbindung der Formel IX

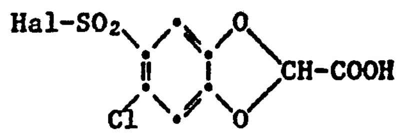

(IX)

oder einem Niederalkylester oder Salz davon, umsetzt, oder

e) zur Herstellung einer Verbindung der Formel I oder eines Salzes dieser Verbindung in einer Verbindung der Formel X,

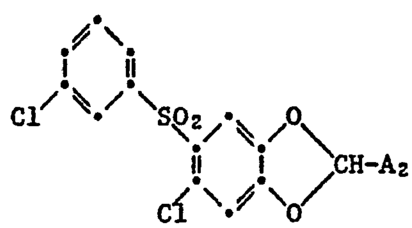

(X)

in welcher $A_2$ eine in die Carboxygruppe überführbare Gruppe bedeutet, die Gruppe $A_2$ in die Carboxygruppe in freier oder Salzform überführt, oder

f) eine Verbindung der Formel XI

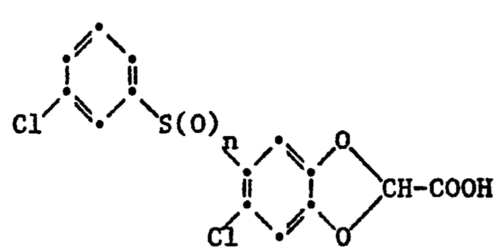

(XI)

in welcher n die Bedeutung von 0 oder 1 hat, zu einer Verbindung der Formel I, in der n die Zahl 2 bedeutet, oxydiert und gegebenenfalls eine erhaltene Verbindung der Formel I in einen Niederalkylester überführt und/oder eine erhaltene Verbindung der Formel I in die optischen Antipoden zerlegt, und/oder eine erhaltene freie Verbindung in ein Salz mit einer Base überführt oder eine solche Verbindung aus einem erhaltenen Salz freisetzt.

11. Behandlung von Oedemen und der Hypertension durch Verabreichung einer prophylaktisch und/oder therapeutisch wirksamen Menge einer Verbindung gemäss einem der Ansprüche 1-4 oder eines pharmazeutisch annehmbaren Salzes einer zur Salzbildung befähigten Verbindung gemäss einem dieser Ansprüche.

12. Die in den Beispielen 1-3 beschriebenen Verfahren.

13. Die nach dem Verfahren des Anspruches 10 erhältlichen Verbindungen.

FO 7.4 HFO/eg*

Patentansprüche für den Vertragsstaat AT

1. Verfahren zur Herstellung des linksdrehenden und rechtsdrehenden Antipoden des Racemates der 5-Chlor-6-(3-chlorbenzolsulfonyl)-1,3-benzodioxol-2-carbonsäure der Formel I

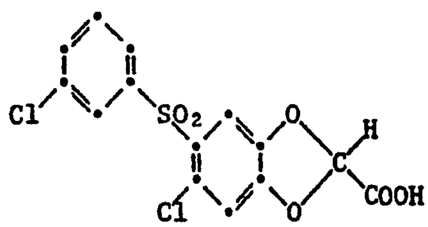

(I)

und deren Salze mit Basen und Niederalkylester des Racemates, dadurch gekennzeichnet, dass man

a) eine Verbindung der Formel II

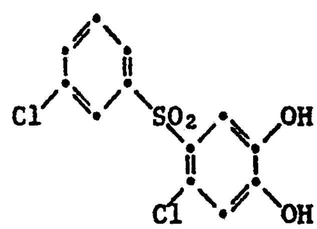

(II)

oder einem Salz davon, mit einer Verbindung der allgemeinen Formel III,

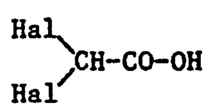

(III)

oder einem Niederalkylester oder einem Salz davon, in welcher Hal Halogen bedeutet, umsetzt, oder

b) in einer Verbindung der Formel IV

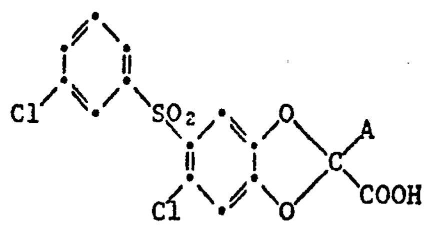

(IV)

einem Niederalkylester oder Salz davon, in welcher A Carboxy, Niederalkoxycarbonyl oder Acetyl bedeutet, den Rest A durch Wasserstoff ersetzt, oder

c) zur Herstellung eines Niederalkylesters einer Verbindung der Formel I eine Verbindung der allgemeinen Formel V

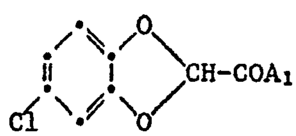

(V)

in welcher $A_1$ Niederalkoxy bedeutet, mit einer Verbindung der Formel VI

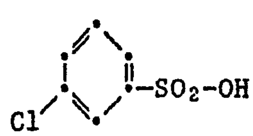

(VI)

oder einem Salz oder Anhydrid desselben umsetzt, oder

d) eine gegebenenfalls in situ gebildete Metallverbindung der allgemeinen Formel VII oder VIII

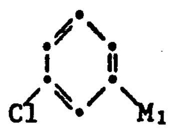 (VII) oder 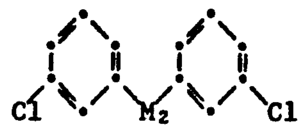 (VIII)

in welcher $M_1$ ein einwertiges bzw. $M_2$ ein zweiwertiges Metallion bedeutet, mit einer Verbindung der Formel IX

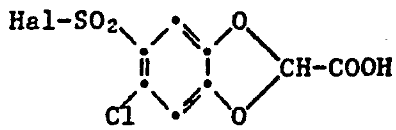 (IX)

oder einem Niederalkylester oder Salz davon, umsetzt, oder

e) zur Herstellung einer Verbindung der Formel I oder eines Salzes dieser Verbindung in einer Verbindung der Formel X,

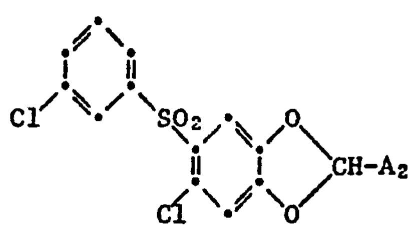 (X)

in welcher $A_2$ eine in die Carboxygruppe überführbare Gruppe bedeutet, die Gruppe $A_2$ in die Carboxygruppe in freier oder Salzform überführt, oder

f) eine Verbindung der Formel XI

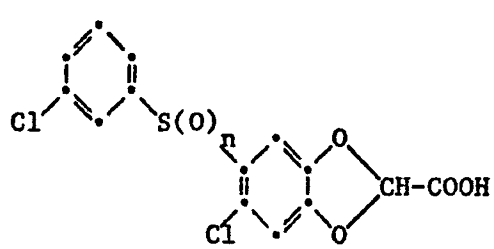 (XI)

in welcher n die Bedeutung von 0 oder 1 hat, zu einer Verbindung der Formel I, in der n die Zahl 2 bedeutet, oxydiert und gegebenenfalls eine erhaltene Verbindung der Formel I in einen Niederalkylester überführt und/oder eine erhaltene Verbindung der Formel I in die optischen Antipoden zerlegt, und/oder eine erhaltene freie Verbindung in ein Salz mit einer Base überführt oder eine solche Verbindung aus einem erhaltenen Salz freisetzt.

2. Verfahren nach Patentanspruch 1, dadurch gekennzeichnet, dass man (-)-5-Chlor-6-(3-chlorbenzolsulfonyl)-1,3-benzodioxol-2-carbonsäure und ihre pharmazeutisch annehmbaren Salze mit Basen herstellt.

3. Verfahren nach Patentanspruch 1, dadurch gekennzeichnet, dass man (+)-5-Chlor-6-(3-chlorbenzolsulfonyl)-1,3-benzodioxol-2-carbonsäure und ihre pharmazeutisch annehmbaren Salze mit Basen herstellt.

4. Verfahren nach Patentanspruch 1, dadurch gekennzeichnet, dass man den 5-Chlor-6-(3-chlorbenzolsulfonyl)-1,3-benzodioxol-2-carbonsäure-äthylester herstellt.

FO 7.4 HFO/eg*